(19) Europäisches Patentamt
European Patent Office
Office européen des brevets

(11) Numéro de publication: **0 002 638**
**B1**

(12) # FASCICULE DE BREVET EUROPEEN

(45) Date de publication du fascicule du brevet: **20.01.82**

(21) Numéro de dépôt: **78400219.8**

(22) Date de dépôt: **07.12.78**

(51) Int. Cl.³: **C 07 C 91/16,**
A 61 K 31/135,
A 61 K 31/36,
C 07 D 317/58

(54) **Sels d'addition d'aralkylamines substituées et composition thérapeutique les contenant.**

(30) Priorité: **13.12.77 GB 5183677**

(43) Date de publication de la demande:
**27.06.79 Bulletin 79/13**

(45) Mention de la délivrance du brevet:
**20.01.82 Bulletin 82/3**

(84) Etats contractants désignés:
**BE CH DE FR GB IT NL SE**

(56) Documents cités:
**DE - A - 1 643 488**
**DE - A - 2 120 203**
**DE - A - 2 303 815**
**GB - A - 1 043 510**
**GB - A - 1 218 135**
**US - A - 2 578 696**

(73) Titulaire: **LABORATOIRE L. LAFON Société anonyme dite:**
**1 rue Georges Médéric**
**F-94701 Maisons-Alfort (FR)**

(72) Inventeur: **Lafon, Louis**
**5 rue de l'Alboni**
**F-75016 Paris (FR)**

(74) Mandataire: **Clisci, Serge et al,**
**CABINET BEAU DE LOMENIE 55 rue d'Amsterdam**
**F-75008 Paris (FR)**

**EP 0 002 638 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Sels d'addition d'aralkylamines substituées et composition thérapeutique les contenant

La présente invention concerne des sels d'addition d'aralkylamines substituées et une composition thérapeutique les contenant.

Les sels d'addition selon l'invention sont des sels d'addition d'acide obtenus à partir d'une aralkylamine substituée de formule:

$$A—CH_2—NH—R \qquad (I)$$

[dans laquelle A représente un groupe 3,4-méthylènedioxyphényle ou $\alpha$-hydroxybenzyle, et R représente $CH(CH_3)_2$ ou $C(CH_3)_3$] et d'un acide organique.

On sait que les bases de formule I sont des produits connus. En particulier, la N-isopropyl-3,4-méthylènedioxybenzylamine (ou N-isopropyl-pipéronylamine) est connue des résumés des *Chemical Abstracts 50,* 4838b et *53,* 8073a, le 2-isopropylamino-1-phényl-1-éthanol est connu de la demande de brevet allemand publiée (OLS) n° 2 052 991 et le 2-tertiobutylamino-1-phényl-1-éthanol est connu du brevet britannique n° 1 043 510.

On sait aussi que les phénylpropanolamines, notamment celles connues de la demande de brevet allemand publiée (OLS) n° 2 120 203, agissent sur le système nerveux central (SNC) mais que leur mode d'action sur le SNC est différent de celui des phényléthanolamines de formule I (où A est un groupe $\alpha$-hydroxybenzyle).

On sait de plus que dans la demande de brevet allemand publiée (OLS) n° 2 303 815 ont été décrits des sels d'addition d'acide (notamment les chlorhydrates et sulfates) de phényléthanolamines différentes de celles de formule I par la présence d'un groupe n-butylamino, iso-butylamino, n-propylamino, au lieu d'un groupe isopropylamino ou tertiobutylamino.

On sait enfin que des sels d'addition d'acide de phényléthanolamines de formule I ont déjà été préconisés en thérapeutique notamment en tant qu'agents antidépresseurs du SNC, à savoir: le chlorhydrate de 2-isopropylamino-1-phényl-1-éthanol, l'acetate et l'hémitartrate de 2-isopropylamino-1-phényl-1-éthanol, l'acetate et l'hémitartrate de 2-tertiobutylamino-1-phényl-1-éthanol (voir l'OLS n° 1 643 488) et le chlorhydrate de 2-tertiobutylamino-1-phényl-1-éthanol (voir le brevet britannique n° 1 043 510).

On vient de trouver de façon surprenante que de nouveaux sels d'addition d'acide de bases de formule I à partir d'acides organiques particuliers sont (i) moins toxiques et (ii) plus actifs sur le SNC en tant qu'agents antidépresseurs que les sels d'addition d'acide les plus proches du point de vue de la structure.

Selon l'invention, on préconise donc un sel d'addition d'aralkylamine substituée, utile notamment en thérapeutique comme agent antidépresseur du SNC, appartenant à la famille des sels d'addition d'acide organique obtenus à partir d'une base libre de formule générale:

$$A—CH_2—NH—R \qquad (I)$$

[dans laquelle A représente un groupe 3,4-méthylènedioxyphényle ou $\alpha$-hydroxybenzyle, et R représente $CH(CH_3)_2$ ou $C(CH_3)_3$] et d'un acide organique, ledit sel d'addition d'aralkylamine étant caractérisé en ce qu'il est choisi parmi l'ensemble constitué par le méthanesulfonate de 2-isopropylamino-1-phényl-1-éthanol, l'hémifumarate et le méthanesulfonate de 2-tertiobutylamino-1-phényl-1-éthanol, et, le propionate et le méthanesulfonate de N-isopropylpipéronylamine.

Les sels d'addition selon l'invention sont préparés selon une méthode connue en soi. Le procédé que l'on préconise consiste à faire réagir la base libre avec un acide, dans des conditions stoechiométriques, dans un solvant, tel que l'éther diéthylique, l'éther diméthylique, l'éthanol ou le méthanol.

Selon l'invention, on préconise une composition thérapeutique utile dans le traitement des maladies du SNC, caractérisée en ce qu'elle renferme en association avec un excipient physiologiquement acceptable au moins un sel d'addition d'acide organique d'aralkylamine substituée de formule I en tant qu'agent antidépresseur du SNC. Les produits préférés sont les sels des exemples 2, 3, 4 et 5.

Préparation I

*Acétate de 2-isopropylamino-1-phényl-1-éthanol* (produit comparatif 1).

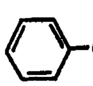 $-CHOH-CH_2-NH-CH(CH_3)_2$ , $CH_3COOH$

n° de code: CRL 40 609 A

a) *2-isopropylamino-1-phényl-1-éthanol.*

On chauffe au reflux pendant 5 h un mélange de 120 g (1 mole) d'oxyde de styrène et de 59 g (1 mole) d'isopropylamine dans 400 ml de méthanol. On évapore à sec et on reprend le résidu d'évaporation par 500 ml d'eau. On filtre la base libre attendue qui a précipité et on lave avec 100 ml d'hexane. On

recristallise la base dans l'hexane, on filtre les cristaux qu'on lave par 500 ml d'hexane et on sèche. On obtient 89 g (rendement: 49%) de 2-isopropylamino-1-phényl-1-éthanol. F = 90°C.

b) *CRL 40 609 A*

On dissout 10 g (0,0558 mole) de la base libre ainsi obtenue dans l'éther. On coule dans cette solution une solution de 3,35 g (0,0558 mole) d'acide acétique dans 50 ml d'éther. On filtre le sel d'addition qui précipite. On lave à l'éther et on obtient 8,4 g (rendement: 62%) de CRL 40 609 A. F = 85°C.

Préparation II

*Propionate de 2-isopropylamino-1-phényl-1-éthanol* (produit comparatif 2).

n° de code: CRL 40 609 B

En procédant comme indiqué dans la préparation Ib, en remplaçant l'acide acétique par de l'acide propionique (4, 13 g; 0,0558 mole), on obtient 8,65 g (rendement: 61%) de CRL 40 609 B. F = 88°C.

Préparation III

*Hémifumarate de 2-isopropylamino-1-phényl-1-éthanol* (produit comparatif 3).

n° de code: CRL 40 609 C

On dissout à chaud dans 67 ml d'éthanol anhydre 12 g (0,0670 mole) de 2-isopropylamino-1-phényl-1-éthanol et 3,886 g (0,0335 mole) d'acide fumarique. On refroidit par un bain de glace et on filtre le précipité formé qu'on lave à l'acétone et sèche. On obtient 14,1 g (rendement: 88%) de CRL 40 609 C. F = 156—158°C.

Analyse % N mesuré : 5,92%
% N théorique : 5,90%

Préparation IV

*Hémisuccinate de 2-isopropylamino-1-phényl-1-éthanol* (produit comparatif 4).

n° de code: CRL 40 609 D

En procédant comme indiqué dans la préparation III, mais en remplaçant l'acide fumarique par de l'acide succinique (3,95 g; 0,0335 mole), on obtient 7,8 g (rendement: 49%) de CRL 40 609 D. F = 125°C.

Analyse : N mesuré : 5,86%
% N théorique : 5,88%

Préparation V

*Méthanesulfonate de 2-isopropylamino-1-phényl-1-éthanol* (exemple 1).

n° de code: CRL 40 609 E

En procédant comme indiqué dans la préparation Ib, en remplaçant l'acide acétique par de l'acide méthanesulfonique, on obtient 13,55 g (rendement: 88%) de CRL 40 609 E. F = 102°C.

Préparation VI

Par réaction de 2-tertiobutylamino-1-phényl-1-éthanol (F = 89—90°C; préparé à partir de 1,5 mole d'oxyde de styrène et 1,84 mole de tertiobutylamine) avec les acides acétique, propionique, fumarique, succinique, méthanesulfonique et citrique, on obtient respectivement les

— acétate (CRL 40 610 A; F = 100°C) — produit comparatif 5 —,
— propionate (CRL 40 610 B; F = 108°C) — produit comparatif 6 —
— hémifumarate (CRL 40 610 C; F = 150°C) — exemple 2 —,
— hémisuccinate (CRL 40 610 D; F = 120°C) — produit comparatif 7 —
— méthanesulfonate (CRL 40 610 E; F = 116—117°C) — exemple 3 — et
— citrate (CRL 40 610 F; F = 116°C — produit comparatif 8 —

de 2-tertiobutylamino-1-phényl-1-éthanol, le citrate ayant pour formule développée

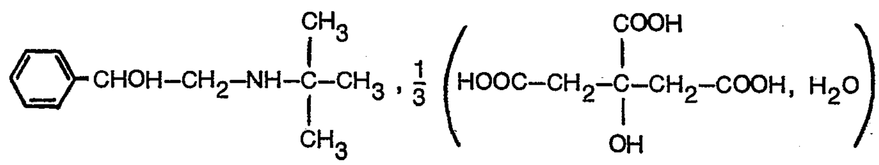

Préparation VII

Par réaction de N-isopropyl-pipéronylamine avec les acides acétique, propionique, fumarique, succinique, méthanesulfonique et citrique, on obtient respectivement les

— acétate (CRL 40 559 A; F = 84°C) — produit comparatif 9 —,
— propionate (CRL 40 599 B; F = 76°C) — exemple 4 —,
— fumarate (CRL 40 599 C; F = 160°C) — produit comparatif 10 —,
— succinate (CRL 40 599 D; F = 125°C) — produit comparatif 11 —,
— méthanesulfonate (CRL 40 599 E; F = 134°C) — exemple 5 —, et
— citrate (CRL 40 599 F; F = 135°C) — produit comparatif 12 —

de N-isopropyl-pipéronylamine.

On a résumé ci-après les résultats des essais pharmacologiques qui ont été entrepris.

a) *Toxicité.*

La DL—50 a été déterminée chez la souris mâle par voie intrapéritonéale. Dans les tableaux I et II ci-après, on a comparé les DL—50 des sels selon l'invention avec celles des produits comparatifs (CP) et des chlorhydrates correspondants.

TABLEAU I
Toxicité des sels de 2-tertiobutylamino-1-phényl-1-éthanol

| Sel | DL—50 du sel (mg/kg) | DL—50 exprimée en base (mg/kg) |
|---|---|---|
| Hémifumarate (exemple 2) | 188 | 145 |
| Méthanesulfonate (exemple 3) | 206 | 138 |
| Acétate (CP 5) | 155 | 118 |
| Propionate (CP 6) | 179 | 129 |
| Hémisuccinate (CP 7) | 157 | 120 |
| Chlorhydrate | 135 | 114 |

TABLEAU II
Toxicité des sels de N-isopropyl-pipéronylamine

| Sel | DL—50 du sel (mg/kg) | DL—50 exprimée en base (mg/kg) |
|---|---|---|
| Propionate (exemple 4) | 235 | 185 |
| Méthanesulfonate (exemple 5) | 252 | 184 |
| Acétate (CP 9) | 190 | 160 |
| Fumarate (CP 10) | 204 | 171 |
| Succinate (CP 11) | 190 | 169 |
| Citrate (CP 12) | 250 | 177 |
| Chlorhydrate | 172 | 135 |

Le tableau I montre que les sels les plus intéressants du point de vue de la toxicité sont les hémifumarate et méthanesulfonate pour les composés I où A est $\alpha$-hydroxybenzyle; le tableau II montre que les sels les plus intéressants sont les méthanesulfonate et propionate pour les composés I où A est 3,4-méthylènedioxyphényle.

b) *Action sur le SNC.*

Administré par voie gastrique et par voie parentérale, le produit de l'exemple 1 (CRL 40 609 E) présente les activités suivantes:

— antagonisme de l'hypothermie réserpinique;
— antagonisme de l'hypothermie et à un degré moindre des tremblements et des signes de stimulation cholinergique périphérique dus à l'oxotrémorine;
— antagonisme de l'hypothermie provoquée par l'apomorphine; et
— diminution de l'agressivité intergroupes (chez la souris mâle).

Administrés par voie gastrique et par voie parentérale, les produits des exemples 2 et 3 (CRL 40 610 C et 40 610 E) présentent le même type d'activité;
— antagonisme de l'hypothermie induite par l'apomorphine sans modifier le comportement de verticalisation et les stéréotypies;
— antagonisme de l'hypothermie due à la réserpine (2,5 mg/kg i.p.) aux doses de 32 à 64 mg/kg chez la sourie [à la dose de 128 mg/kg, cet effet antagoniste n'est plus net]; et
— antagonisme de l'hypothermie induite par l'oxotrémorine, sans modifier les tremblements et les symptômes cholinergiques périphériques (salivation, lacrymation, défécation).

Dans le tableau III ci-après, on a consigné les résultats obtenus avec le CRL 40 610 C (exemple 2) administré par voie orale 30 minutes avant administration d'apomorphine (lots de 6 animaux par dose; 12 témoins).

TABLEAU III

Antagonisme du produit de l'exemple 2 vis-à-vis de l'apomorphine

| Produit | Dose (mg/kg) | Température (°C) au temps | | | Verticalisation T = +25 min | Stéréotypies T = +25 min |
|---|---|---|---|---|---|---|
| | | T = -30 min | T = 0 | T = +30 min | | |
| – | (témoin) | 36,1 | 37,0 | 33,1 | 1,75 | 2,75 |
| CRL 40 610 C | 1 | 35,8 | 36,9 | 32,8 | 1,83 | 3,00 |
| CRL 40 610 C | 4 | 36,2 | 37,3 | 34,2 | 1,67 | 2,67 |
| CRL 40 610 C | 16 | 36,1 | 36,9 | 35,3 | 1,67 | 2,50 |
| CRL 40 610 C | 64 | 36,3 | 36,8 | 35,8 | 2,00 | 2,83 |

Les produits des exemples 4 et 5 (CRL 40 599 B et CRL 40 599 E) présentent un antagonisme de l'hypothermie induite par l'apomorphine, l'oxotrémorine et la réserpine. A fortes doses, ils potentialisent les stéréotypies de l'amphétamine et diminuent l'agressivité.

L'ensemble de ces résultats montre que les sels selon l'invention sont des agents antidépresseurs du SNC.

Ces sels sont administrables chez l'homme par voie orale comme agents antidépresseurs, alors que, en général, les antagonistes $\beta$-sympathomimétiques qui sont voisins du point de vue de la structure doivent être utilisés par voie parentérale.

**Revendications**

1. Sel d'addition d'aralkylamine substituée, utile notamment en thérapeutique comme agent antidépresseur du SNC, appartenant à la famille des sels d'addition d'acide organique obtenus à partir d'une base libre de formule générale:

$$A—CH_2—NH—R \qquad (I)$$

[dans laquelle A représente un groupe 3,4-méthylènedioxyphényle ou $\alpha$-hydroxybenzyle, et R représente $CH(CH_3)_2$ ou $C(CH_3)_3$] et d'un acide organique, ledit sel d'addition d'aralkylamine étant caractérisé en ce qu'il est choisi parmi l'ensemble constitué par le méthanesulfonate de 2-isopropylamino-1-phényl-1-éthanol, l'hémifumarate et le méthanesulfonate de 2-tertiobutylamino-1-phényl-1-éthanol, et, le propionate et le méthanesulfonate de N-isopropylpipéronylamine.

2. Composition thérapeutique, caractérisée en ce qu'elle renferme, en association avec un excipient physiologiquement acceptable, au moins un sel d'addition selon la revendication 1.

**Claims**

1. A substituted aralkylamine addition salt, for use in particular in therapy as an antidepressant of the central nervous system, belonging to the family of organic acid addition salts obtained from a free base of general formula:

$$A—CH_2—NH—R \quad (I)$$

where A is a 3,4-methylenedioxyphenyl or $\alpha$-hydroxybenzyl group, and R is $CH(CH_3)_2$ or $C(CH_3)_3$, and from an organic acid, the said aralkylamine addition salt being characterised in that it is selected from the group consisting of 2-isopropylamino-1-phenyl-1-ethanol methanesulfonate, 2-tertiobutylamino-1-phenyl-1-ethanol hemifumarate and methanesulfonate, and N-isopropylpiperonylamine methanesulfonate and propionate.

2. A pharmaceutical composition, characterised in that it contains, in combination with a physiologically acceptable excipient, at least one addition salt according to claim 1.

**Patentansprüche**

1. Additionssalz von substituiertem Aralkylamin, welches in Therapeutik vorteilhaft ist, und insbesondere auf das Zentralnervensystem als Antidepressivum einwirkt, und welches zu der Familie der Säureadditionssalze gehört, die aus einer freien Base der allgemeinen Formel:

$$A—CH_2—NH—R \quad (I)$$

[worin A 3,4-Methylendioxyphenyl oder $\alpha$-Hydroxybenzyl bedeutet und R $CH(CH_3)_2$ oder $C(CH_3)_3$ bedeutet] und aus einer organischen Säure erhalten werden, dadurch gekennzeichnet, dass es aus der Gruppe, die aus Methansulfonat von 2-Isopropylamino-1-phenyl-1-äthanol, Hemifumarat und Methansulfonat von 2-Tertiobutylamino-1-phenyl-1-äthanol und Propionat und Methansulfonat von N-Isopropyl-piperonylamin besteht, gewählt wird.

2. Therapeutische Zusammensetzung, dadurch gekennzeichnet, dass sie, in Kombination mit einem physiologisch verträglichen Hilfsstoff, mindestens ein Additionssalz nach Anspruch 1 enthält.